# EUROPÄISCHE PATENTANMELDUNG

(11) **EP 3 081 176 A1**
(43) Veröffentlichungstag der Anmeldung: **19.10.2016**
(21) Anmeldenummer: 15001063.5
(22) Anmeldetag: 14.04.2015
(51) Int. Cl.: A61B 17/16, A61C 8/00, A61B 17/14, A61C 1/05, A61B 17/00

(54) **WERKZEUG ZUR EINBRINGUNG EINER HELIKALEN KAVITÄT IN EINEN KNOCHEN**

(71) Anmelder: Teichmann, Gernot, 40667 Meerbusch (DE)
(72) Erfinder: Teichmann, Gernot, 40667 Meerbusch (DE)
(74) Vertreter: Cohausz Hannig Borkowski Wißgott

(57) **Zusammenfassung**

Die Erfindung betrifft ein chirurgisches Werkzeug (1) zur Einbringung einer helikalen Kavität in einen Knochen mit einem zumindest einen Teil einer Antriebswelle (9) aufnehmenden Hauptteil (2) und einem an diesem gehaltenen Werkzeugteil (3), welches ein Funktionselement (5) umfasst, das durch Ausübung einer oszillierenden Bewegung die Kavität im Knochen erzeugt. Das Werkzeugteil (3) umfasst einen abstehenden, drehbaren Schaft (4), an dessen dem Hauptteil (2) gegenüberliegenden Ende sich ein das Funktionselement bildender Stift (5) durch eine Öffnung (8) aus dem Schaft (4) heraus erstreckt, der zur Ausübung einer Materialabtragung des Knochens in seiner Längsrichtung oszillierbar ist. Der Schaft (4) ist derart mit der Antriebswelle (9) gekoppelt, dass diese zumindest mittelbar ein Drehmoment auf den Schaft (4) und den Stift (5) ausübt, wobei vom Schaft (4) zumindest ein Flügel (6) radial absteht, der in einem Abstand hinter dem Stift (5) beginnend sich in Umfangsrichtung des Schafts (4) helikal in Richtung des Hauptteils (2) erstreckt. Die Antriebswelle (9) weist einen von ihrem stirnseitigen Ende abstehenden Exzenterstift (11) auf, der mit einer sich durch den Schaft (4) erstreckenden Vibrationswelle (7) in Eingriff steht, wobei der Stift (5) an der Vibrationswelle (7) fest gehalten ist.

## Beschreibung

Die vorliegende Erfindung betrifft ein chirurgisches, insbesondere ein kieferchirurgisches Werkzeug zur Einbringung einer helikalen Kavität in einen Knochen, insbesondere einen Kieferknochen mit einem zumindest einen Teil einer Antriebswelle aufnehmenden Hauptteil und einem an diesem gehaltenen Werkzeugteil, welches ein Funktionselement umfasst, das durch Ausübung einer oszillierenden Bewegung die Kavität im Knochen erzeugt. Das Werkzeugteil umfasst einen abstehenden, drehbaren Schaft, an dessen dem Hauptteil gegenüberliegenden Ende sich ein das Funktionselement bildender Stift durch eine Öffnung aus dem Schaft heraus erstreckt, der zur Ausübung einer Materialabtragung des Knochens in seiner Längsrichtung oszillierbar ist, wobei der Schaft derart mit der Antriebswelle gekoppelt ist, dass diese zumindest mittelbar ein Drehmoment auf den Schaft und den Stift ausübt, und wobei vom Schaft zumindest ein Flügel radial absteht, der in einem Abstand hinter dem Stift beginnend sich in Umfangsrichtung des Schafts helikal in Richtung des Hauptteils erstreckt.

Für die Verankerung zahntechnischer Implantate in einem Kieferknochen ist es erforderlich, eine Kavität vorzusehen, die das Implantat respektive einen dieses tragende Implantatkörper aufnimmt. Im Stand der Technik sind diverse Ausführungsvarianten zahntechnischer Implantate bekannt. Insbesondere ist ein Werkzeug der genannten Gattung aus der deutschen Patentanmeldung DE 10 2006 057 019 A1 bekannt. Neben der Kieferchirurgie sind aber auch in anderen chirurgischen Bereichen Implantate in Knochen erforderlich.

Eine Variante dieser Implantate besitzt einen Verankerungskörper, der aus einem massiven zylindrischen Mittelteil und zumindest einem von diesem radial abstehenden, sich schraubenlinienförmig um den Umfang des Mittelteils herum erstreckenden Flügel besteht. Auch sind zwei oder sogar mehr solcher Flügel möglich, wobei dann die Steigung der jeweiligen Flügel erheblich steiler ist als bei Vorhandensein eines einzigen Flügels. Das Implantieren eines derartigen Verankerungskörpers im Knochen kann auf unterschiedliche Art und Weise erfolgen. Gemeinsam haben diese Implantierungsarten, dass zunächst eine Bohrung in den Knochen eingebracht werden muss, die zumindest das zylindrische Mittelteil aufnimmt.

Beispielsweise kann gemäß einer Variante der Durchmesser der Bohrung gleich dem Außendurchmesser des Mittelteils sein, wobei der oder die Flügel des Implantatkörpers dann selbstschneidend sind und der Implantatkörper in der Art einer Schraube in den Knochen eingedreht wird, wobei sich die Flügel wie die Gewinde einer Schraube in den Knochen hineinschneiden. Dies ist jedoch nur unter sehr starker Kraft- bzw. Drehmomentaufwendung möglich. Im Falle eines Zahnimplantats ist ein derartiges Einschrauben des Implantatkörpers für den Patienten äußerst unangenehm.

Eine Alternative besteht darin, die Bohrung derart groß auszugestalten, dass ihr Durchmesser dem Gesamtdurchmesser des Implantatkörpers, d.h. einschließlich der radialen Breite des Flügels entspricht. In diesem Fall wird der Implantatkörper lediglich in die Bohrung eingeschoben, ohne dass sich die Flügel in den Knochen schneiden. Ein Nachteil dieser Variante ist jedoch, dass viel Knochenmaterial entfernt werden muss und es sehr lange dauert, bis das Knochenmaterial an den Implantatkörper heran gewachsen ist. Der Heilungsprozess ist damit unverhältnismäßig lang.

Es ist natürlich gemäß einer dritten Variante auch möglich, den Durchmesser der Bohrung etwas geringer als den Gesamtdurchmesser des Implantatkörpers zu wählen, so dass sich beim Einsetzen des Implantatkörpers der oder die Flügel zwar in den Knochen hinein schneiden, jedoch die Eintauchtiefe der Flügel nur minimal ist, beispielsweise 0,5 bis 1 mm beträgt. Wenngleich der Implantatkörper bei dieser Variante schon bei seinem Einsetzen fest im Knochen einliegt, ist der Heilprozess nach wie vor lang, da ein großer Volumenbereich wieder mit Knochenmaterial zuwachsen muss.

Es ist daher Aufgabe der vorliegenden Erfindung, ein chirurgisches Werkzeug bereitzustellen, um eine Kavität in einen Knochen, insbesondere einen Kieferknochen einzubringen, deren Form korrespondierend der helikalen Form des Flügels eines Implantatkörpers ist, so dass nur eine minimale Menge an Knochenmaterial für das Einsetzen des Implantatkörpers aus dem Knochen entfernt werden muss und der Heilungsprozess damit erheblich verkürzt wird.

Diese Aufgabe wird durch ein chirurgisches Werkzeug gemäß Anspruch 1 gelöst. Vorteilhafte Weiterbildungen dieses Werkzeugs sind in den Unteransprüchen angegeben.

Erfindungsgemäß wird ein chirurgisches, insbesondere kieferchirurgisches Werkzeug zur Einbringung einer helikalen Kavität in einen Knochen, insbesondere einen Kieferknochen mit einem zumindest einen Teil einer Antriebswelle aufnehmenden Hauptteil und einem an diesem gehaltenen Werkzeugteil vorgeschlagen, welches ein Funktionselement umfasst, das durch Ausübung einer oszillierenden Bewegung die Kavität im Knochen erzeugt, wobei das Werkzeugteil einen abstehenden, drehbaren Schaft umfasst, an dessen dem Hauptteil gegenüberliegenden Ende sich ein das Funktionselement bildender Stift durch eine Öffnung aus dem Schaft heraus erstreckt, der zur Ausübung einer Materialabtragung des Knochens in seiner Längsrichtung oszillierbar ist. Der Schaft ist derart mit der Antriebswelle gekoppelt, dass diese zumindest mittelbar ein Drehmoment auf den Schaft und den Stift ausübt, wobei vom Schaft zumindest ein Flügel radial absteht, der in einem Abstand hinter dem Stift beginnend sich in Umfangsrichtung des Schafts helikal in Richtung des Hauptteils erstreckt, wobei die Kopplung des Schafts mit der Antriebswelle über eine hydrodynamische Kupplung erfolgt.

Ein Kernaspekt des erfindungsgemäßen Werkzeugs ist es, dass das Werkzeugteil während des bestimmungsgemäßen Betriebs zwei Bewegungen vollführt, nämlich zum einen eine translatorische Bewegung, um die Materialabtragung am Knochen durch den Stift zu bewirken, und zum anderen eine rotatorische Bewegung, um den Stift voranzutreiben. Unter einer rotatorischen Bewegung oder Drehbewegung des Werkzeugteils, Schafts oder Stifts ist hier lediglich ein Vorschub entlang eines Kreissegments zu verstehen, d.h. es sind keine vollen Umdrehungen gemeint. Auf den Schaft respektive den Stift wird vielmehr lediglich ein Drehmoment ausgeübt, das einen Vorschub des Stifts in Umfangsrichtung bewirkt, so dass sich das Werkzeugteil zunehmend in den Knochen hineinarbeitet.

Durch die Überlagerung der beiden Bewegungen entsteht in dem Knochen die gewünschte Kavität, die sich schließlich helikal in den Knochen hineinerstreckt. Der vom Schaft radial abstehende Flügel dient dabei der Stabilisierung und Führung des Werkzeugteils.

Durch den Flügel erhält der Schaft eine Form, die im Wesentlichen dem zu implantierenden Implantatkörper entspricht. Der zumindest eine Flügel tritt mit zunehmendem Fortschritt der Kavitätsherstellung in die Kavität ein und bildet somit eine Führung für das Werkzeug bzw. das Werkzeugteil. Ferner definiert der Flügel den Winkel, in dem sich der Stift in den Kieferknochen hineinarbeitet, so dass sichergestellt ist, dass die Kavität mit dem Schraubengewinde des zu implantierenden Implantatkörpers korrespondiert.

Die Erstreckung des Flügels in radialer Richtung entspricht maximal dem Maß, um das der Stift vom Schaft hervorsteht, damit der Flügel keinen Widerstand bildet, wenn das Werkzeug in den Kieferknochen hinein getrieben wird. Denn dies würde das für den Vorschub aufzubringende Drehmoment erheblich erhöhen. Aus diesem Grund ist auch die Dicke des Flügels geringer als die Dicke des Stifts. Beispielsweise kann die Dicke des Flügels zwischen 0,5 und 1,2mm, insbesondere 1 mm betragen. Die Dicke des Stifts ist dann entsprechend größer zu wählen. Da der zumindest eine Flügel die Außenwand der Kavität nicht berührt und der Stift in der Bewegungsrichtung des Schafts dem Flügel vorgelagert ist, beteiligt sich der Flügel nicht aktiv an der Herstellung der Kavität. Er muss also nicht selbstschneidend ausgeführt sein und kann folglich eine stumpfe Außenkante besitzen. Dies hat den Vorteil, dass diese Kante nicht speziell bearbeitet sein muss.

Es genügt, wenn sich der Flügel entlang 2/3 bis 4/5 des Schaftumfangs erstreckt. Selbst wenn das in die Kavität einzusetzende Implantat einen Flügel mit einer vollen oder gar 1 ½-fachen Schraubenumdrehung um das Mittelteil des Implantatkörpers herum besitzt, kann der Flügel des erfindungsgemäßen Werkzeugs gleichwohl eine geringere Umfangslänge aufweisen, weil dieser Flügel nur zur Stabilisierung und Führung des Werkzeuges respektive des Stifts dient.

Der Flügel kann in radialer Richtung eine Breite aufweisen, die das 0,5- bis 1,1-fache des Durchmessers des Schafts besitzt. Entsprechend steht der Stift vom Schaft um eine Länge hervor, die dem 0,6- bis 1,2-fachen des Schaftdurchmessers entspricht. So können beispielsweise bei einem Schaftdurchmesser von 3mm der Gesamtdurchmesser von Schaft und Flügel 9mm betragen. Je nach zu implantierendem Implantatkörper kann jedoch auch eine geringere oder größere Erstreckung des Flügels respektive des Stifts in radialer Richtung realisiert werden.

Es sei angemerkt, dass vor der Verwendung des erfindungsgemäßen chirurgischen Werkzeugs ebenfalls eine Bohrung in den Knochen eingebracht werden muss, deren Durchmesser mindestens, vorzugsweise genau dem Außendurchmesser des Schafts entspricht. Sodann kann das erfindungsgemäße Werkzeug auf den Knochen aufgesetzt werden, wobei der Stift am Knochen zur Anlage gelangt. Zur Stabilisierung der Position kann die Öffnung, durch die sich der Stift hindurch erstreckt, in einem geringen Abstand von beispielweise 1 bis 2 mm von dem dem Hauptteil gegenüberliegenden Ende des Schafts vorhanden sein, so dass der Schaft mit seinem bis zur Öffnung reichenden Endabschnitt in die Bohrung im Knochen eingesetzt werden kann, bevor das Werkzeug in Betrieb genommen wird. Er ist damit in der Bohrung gehalten und laterale Bewegungen des Werkzeugteils werden verhindert.

Wie bereits beschrieben, vollführt der Stift im Betrieb des erfindungsgemäßen Werkzeugs eine Oszillationsbewegung in Längsrichtung sowie gleichzeitig eine rotatorische Bewegung. Die hierfür jeweils benötigten Antriebsmittel können beliebig ausgebildet sein. Es können unabhängige Antriebsmittel, vorzugsweise jedoch ein Antriebsmittel vorgesehen werden, das beide Bewegungen bewirkt. Dies wird nachfolgend noch veranschaulicht.

Zumindest ein Teil der Oberfläche des Stifts kann beispielsweise diamantiert sein, so dass der Stift infolge der Bewegung wie eine Feile wirkt, die Knochenmaterial wegfeilt. Alternativ kann zumindest ein Teil der Oberfläche des Stifts ein Sägezahnprofil, insbesondere ein rotationssymmetrisches Sägezahnprofil aufweisen, so dass der Stift infolge der Bewegung wie eine Säge wirkt, die Knochenmaterial wegsägt.

Die Oszillation kann beispielsweise bei einer Frequenz zwischen 25 kHz und 30 kHz, insbesondere bei ca. 28 kHz erfolgen, wobei im Falle eines sägenden Stift auch eine deutlich geringere Frequenz möglich ist, beispielsweise zwischen 50 und 600Hz.

Im Falle der feilenden Wirkung des Stifts genügt es, wenn die Auslenkung des Stifts mikroskopisch ist, d.h. 0,1 mm oder weniger beträgt. Der Stift arbeitet dabei nicht unter Druck, jedoch zwecknotwendigerweise unter Kontakt mit dem Kieferknochen.

Die Oszillation kann beispielsweise mittels eines Vibrationsantriebs erreicht werden, der mit einer sich durch den Schaft erstreckenden Vibrationswelle gekoppelt ist, an deren Ende der Stift fest gehalten ist. Die Vibrationswelle dient zur Übertragung der Vibrationen vom Antrieb zum Stift, da der Vibrationsantrieb aus Platzgründen nicht im Schaft angeordnet werden kann. Er kann jedoch im Hauptteil oder außerhalb des erfindungsgemäßen Werkzeugs angeordnet sein.

Der Vibrationsantrieb kann beispielsweise ein Piezoelement sein, das bei entsprechender Anregung mit einer Wechselspannung Schwingungen mikroskopischer Auslegungen gleicher Frequenz erzeugt. Die Schwingungen können dabei eine Amplitude bis zu einigen 100 µm betragen.

Alternativ kann der Vibrationsantrieb durch einen Miniaturmotor oder eine spezielle Welle gebildet sein, die eine Unwucht aufweist oder trägt, welche die Vibrationen erzeugt. Durch das Drehen der Unwucht wird der Motor respektive die Welle in Schwingung versetzt. Durch eine mechanische Kopplung des Elektromotors bzw. der Welle mit der Vibrationswelle werden diese Schwingungen auf die Vibrationswelle übertragen.

Gemäß einer anderen, bevorzugten Ausführungsvariante kann die Oszillation auch durch die Antriebswelle erreicht werden. Dies hat den Vorteil, dass kein gesonderter Vibrationsantrieb benötigt wird. Hierzu kann die Antriebswelle einen von ihrem stirnseitigen Ende abstehenden Exzenterstift aufweisen, der mit der sich durch den Schaft erstreckenden Vibrationswelle in Eingriff steht. Dieser Eingriff erfolgt geeigneterweise derart, dass der Exzenterstift in eine längliche Nut hineinragt, die in der Stirnseite der Vibrationswelle ausgebildet ist. Der Exzenterstift ist ein Vorsprung, der bezogen auf die Achse der Antriebswelle außermittig liegt. Bei einer Drehung der Welle vollführt er folglich eine Kreisbewegung. Durch den Eingriff des Exzenterstifts in die längliche Nut wirkt dieser als Mitnehmer, der seine Kreisbewegung in eine eindimensionale Hin-und-Her-Bewegung der Vibrationswelle überträgt. Eine Vibration der Vibrationswelle wird folglich durch die Drehung der Antriebswelle erreicht, die beispielsweise zwischen 20.000 und 40.000 Umdrehungen pro Minute betragen kann.

Diese endseitig der Vibrationswelle erzeugten Vibrationen werden dann von der Vibrationswelle an ihr gegenüberliegendes Ende übertragen, an dem der Stift fest gehalten ist. Hierdurch führt auch der Stift die Oszillationsbewegung durch.

Die Vibrationswelle kann nicht nur als Überträger der Vibration vom Antrieb zum Stift sondern kann auch als Verstärker oder Dämpfer der Vibrationen dienen. Letzteres kann beispielsweise durch die Lage eines Festlagers beeinflusst werden, das im Schaft angeordnet ist und gegen das sich die Vibrationswelle abstützen kann. Vorzugsweise ist das Festlager eine Scheibe, durch die sich die Vibrationswelle idealerweise konzentrisch hindurch erstreckt. Ist dieses Festlager beispielsweise mittig zwischen dem Ort der Vibrationseinkopplung oder Erzeugung und dem Stift angeordnet, so findet weder eine Verstärkung noch eine Dämpfung statt. Wird das Lager hingegen ausgehend von dieser Mitte näher zur Erregerquelle verschoben, wirkt der Stab bzw. die Vibrationswelle in Folge des größeren Abstandes zwischen Festlager und Stift gegenüber dem Abstand zwischen Festlager und Erregungsquelle als Verstärker und die Vibrationen sind am Ende der Vibrationswelle, d.h. dort, wo der Stift gehalten ist, größer als an ihrem Anfang, an dem der Vibrationsantrieb die Schwingungen in die Vibrationswelle einkoppelt oder der Exzenterstift in die Langnut eingreift.

Vorzugsweise kann die Lage des Festlagers zumindest in einem begrenzten Maße in axialer Richtung zur Vibrationswelle veränderlich sein, um je nach gewünschter Oszillation des Stifts einen mehr oder weniger großen Bewegungshub des Stiftes zu erreichen.

Neben der Art und Weise, die Oszillation des Stifts zu erzeugen, kann auch sein Vorschub, d.h. die Kopplung von Schaft und Antriebswelle auf vielerlei Art und Weise erreicht werden.

Erfindungsgemäß erfolgt die Kopplung des Schafts mit der Antriebswelle über eine hydrodynamische Kupplung. Bei einer derartigen Kupplung erfolgt die Kraftübertragung von einem treibenden Element zu einem getriebenen Element mittels einer Flüssigkeit, insbesondere die Bewegungsenergie dieser Flüssigkeit. Die Flüssigkeit wird von dem treibenden Element in Rotation versetzt, so dass ein Wirbel in der Art eines Zyklons entsteht, welcher wiederum das getriebene Element mitnimmt. Zum Bewegen der Flüssigkeit kann das treibende Element Lamellen oder Schaufeln aufweisen, die in Rotation versetzt werden und die Flüssigkeit antreiben. Entsprechend kann das getriebene Element ebenfalls Lamellen oder Schaufeln aufweisen, welche durch das rotierende Wasser mitgenommen und so das getriebene Element ebenfalls in Rotation versetzt wird.

Als treibendes Element kann beispielsweise die Antriebswelle dienen, die an ihrem im Hauptteil liegenden Ende vorzugsweise radial oder tangential nach außen abstehende Lamellen oder Schaufeln aufweist. Entsprechend kann als angetriebenes Element das Hauptteil dienen, das an seiner Innenseite radial oder tangential nach innen gerichtete Lamellen oder Schaufeln aufweist. Diese können den Lamellen oder Schaufeln der Antriebswelle gegenüberliegen. Vorzugsweise sind die Lamellen des treibenden und angetriebenen Elements im Falle einer tangentialen Anordnung gegensätzlich zueinander tangential angeordnet bzw. im Falle von Schaufeln gegensätzlich zueinander gebogen, so dass jeweils ein hohes Drehmoment auf die Flüssigkeit auf die Flüssigkeit übertragen bzw. von dieser aufgenommen wird.

Durch die hydrodynamische Kupplung wird folglich über eine Flüssigkeit ein Drehmoment von der Antriebswelle auf das Hauptteil übertragen. Dabei kann das Hauptteil ein zylindrisches Gehäuse aufweisen, das drehbar gelagert ist. Das Werkzeugteil kann drehfest mit dem Hauptteil verbunden sein, so dass das Werkzeugteil und folglich auch der Schaft samt Stift durch die Drehung des Hauptteils mitgedreht wird bzw. das Drehmoment auch auf das Werkzeugteil samt Schaft und Stift übertragen wird.

Beispielsweise kann das Werkzeugteil das zylindrische Gehäuse trommelartig umgreifen und an diesem fest gehalten sein, so dass es die notwendige Stabilität erhält. Hierzu kann das Werkzeugteil entsprechend einen rohrförmigen Abschnitt aufweisen, der konzentrisch zum Hauptteil liegt und an einem axialen Ende mittels einer Lochscheibe geschlossen ist. Des Weiteren kann der Schaft einteilig mit diesem Abschnitt ausgebildet sein, wobei er koaxial von diesem auf der dem Hauptteil gegenüberliegenden Seite absteht, so dass die Lochscheibe den Abstand zwischen der Außenseite des Schafts und der Innenseite des rohrförmigen Abschnitts schließt.

Sofern sich eine Vibrationswelle durch den Schaft hindurch erstreckt, ist der Schaft entsprechend hohl ausgebildet. Beispielsweise kann der Schaft durch ein dünnwandiges Rohr gebildet sein. Ein hohler Schaft hat den Vorteil, dass durch ihn hindurch eine Flüssigkeit, beispielsweise sterilisiertes Wasser, gefördert werden kann. Idealerweise besitzt der Schaft an seinem dem Hauptteil abgewandten Ende eine Öffnung, durch die die Flüssigkeit austreten und die Kavität an der Arbeitsfront ausspülen kann. Abgeriebene Knochenpartikel werden dabei mitgenommen und aus der bereits erstellten Kavität heraus geschwemmt. Die Öffnung kann beispielsweise an der Stirnseite des Schafts vorhanden sein, so dass die Flüssigkeit in axialer Richtung zum Schaft aus diesem strömt. Die Flüssigkeit kann alternativ oder zusätzlich auch radial durch einen Ringspalt derjenigen Öffnung austreten, durch den sich der Stift aus dem Schaft heraus erstreckt. Dies hat den Vorteil, dass die Flüssigkeit direkt in die bereits erzeugte helikale Kavität, d.h. an die Arbeitsfront strömt, so dass abgetragenes Knochenmaterial, das die Oberflächenstrukturierung des Stifts zusetzt, heraus gespült und abtransportiert wird. Ferner kann neben dem Stift auf der dem Flügel gegenüberliegenden Seite in derselben Radialebene des Schafts wie der Stift eine weitere Öffnung im Schaft vorhanden sein, durch die die Flüssigkeit strömt.

Um die Ausschwemmung abgeriebener Knochenpartikel weiter zu verbessern, kann der Stift hohl ausgebildet sein, wobei er an seinem im Schaft einliegenden Endbereichen Querbohrungen aufweisen kann, durch die die in den Schaft geförderte Spülflüssigkeit in den Stift eintreten kann. An dem der Vibrationswelle gegenüberliegenden Ende kann der Stift dann offen ausgebildet sein, so dass die Spülflüssigkeit in axialer Richtung aus diesem Ende des Stifts austreten und dann achsparallel vom radialen Ende der hergestellten Kavität in Richtung des Schafts strömen kann. Die abgeschabten Knochenpartikel werden somit effektiv heraus gespült.

Damit der Schaft nicht am Boden der Bohrung im Knochen vollflächig mit seiner Stirnseite aufliegt, kann die Stirnseite uneben ausgebildet sein. So kann sie beispielsweise einer bogen-, insbesondere sinusförmigen Linie entsprechen, so dass der Schaft nur an zwei oder mehr Stellen in der Knochenbohrung aufliegt.

Gemäß einer vorteilhaften Weiterbildung des erfindungsgemäßen chirurgischen Werkzeugs, kann dem Stift diametral gegenüberliegend ein zweiter Stift angeordnet sein, der sich ebenfalls durch eine Öffnung aus dem Schaft heraus erstreckt und der zur Ausübung einer Feilwirkung in Längsrichtung oszillierbar ist. Sofern dieser Stift durch dieselbe Quelle in Schwingung versetzt wird, wie der erste Stift, wird der zweite Stift im Gegentakt zum ersten Stift bewegt. Durch die Verwendung von zwei oszillierenden Stiften kann an gegenüberliegenden Stellen im Knochen gleichzeitig eine helikale Kavität erzeugt werden. Dies ist für Implantatkörper erforderlich, die zwei um 180° versetzt zueinander liegende Flügel in der Art von zwei Gewindegängen aufweisen.

Des Weiteren kann vom Schaft ein zweiter Flügel radial abstehen, der um 180° versetzt zum ersten Flügel liegt und sich in einem Abstand hinter dem zweiten Stift beginnend in Umfangsrichtung des Schafts helikal nach oben erstreckt. Auf diese Weise wird ein 180° symmetrischer Aufbau des Schafts erreicht. Auch der zweite Flügel dient lediglich zur Führung und Bewegungsstabilisierung des Werkzeugs, sobald der zweite Stift die Ausbildung der entsprechenden Kavität soweit voran getrieben hat, dass sein nachgelagerter Flügel in diese Kavität zunehmend eingreift. Auch der zweite Flügel steht radial gegenüber dem zweiten Stift zurück und kann daher an seiner Peripherie stumpf ausgebildet sein.

Da die Oberfläche des Stifts mit der Zeit verschleißen kann, ist es erforderlich, den Stift bzw. die Stifte auswechselbar vorzusehen. Hierfür kann der erste und/oder der zweite Stift mittels eines Schraubgewindes an der Vibrationswelle befestigt sein. Es ist jedoch alternativ auch möglich, das gesamte Werkzeugteil als Verschleißteil auszuwechseln. Hierzu ist vorgesehen, dass das Werkzeugteil lösbar an dem Hauptteil befestigt, insbesondere aufgesteckt ist.

Der erste und/oder der zweite Stift können im Querschnitt rund oder oval sein, wobei ihre Dicke mindestens so dick, vorzugsweise 10 bis 20% dicker als die Dicke des bzw. der Flügel sein sollte, damit der bzw. die Flügel in der gebildeten Kavität nicht stecken bleiben. Ein Steckenbleiben ist grundsätzlich möglich, wenn die Dicke des Stifts und die Dicke des Flügels im Wesentlichen gleich groß sind und sich von dem Stift abgeriebene Knochenpartikel zwischen den Flügel und den Knochen setzen.

Weitere Vorteile und Merkmale der Erfindung werden nachfolgend anhand eines Ausführungsbeispiels und der beigefügten Figuren näher erläutert. Es zeigen:
- Figur 1:: schematische Darstellung eines erfindungsgemäßen kieferchirurgischen Werkzeugs von der Seite
- Figur 2:: schematische Darstellung des kieferchirurgischen Werkzeugs von unten
- Figur 3:: Prinzipskizze eines erfindungsgemäßen kieferchirurgischen Werkzeugs in geschnittener Darstellung
- Figur 4:: Prinzipdarstellung der Kopplung zwischen Vibrationswelle und Exzenterstift
- Figur 5:: Stift mit Sägezahnprofil

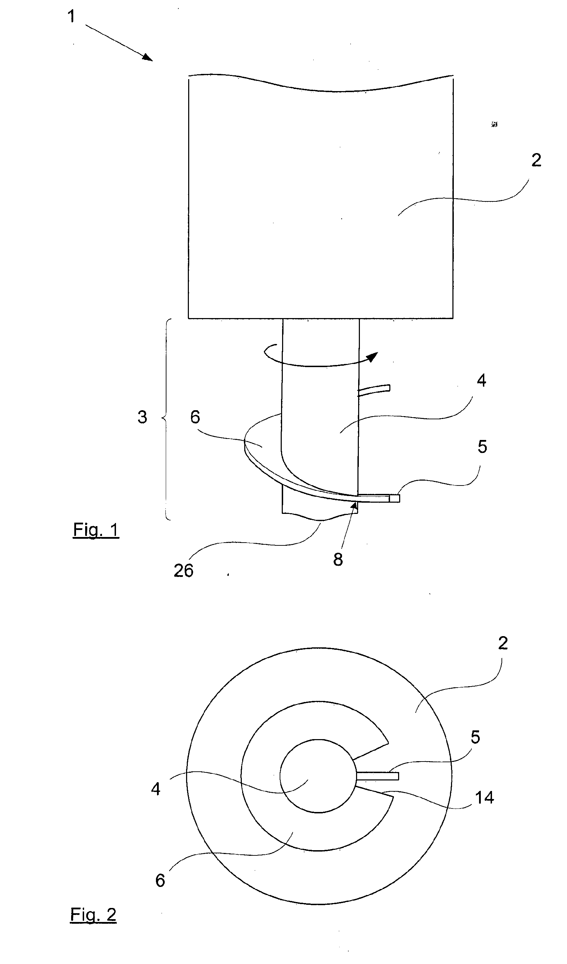

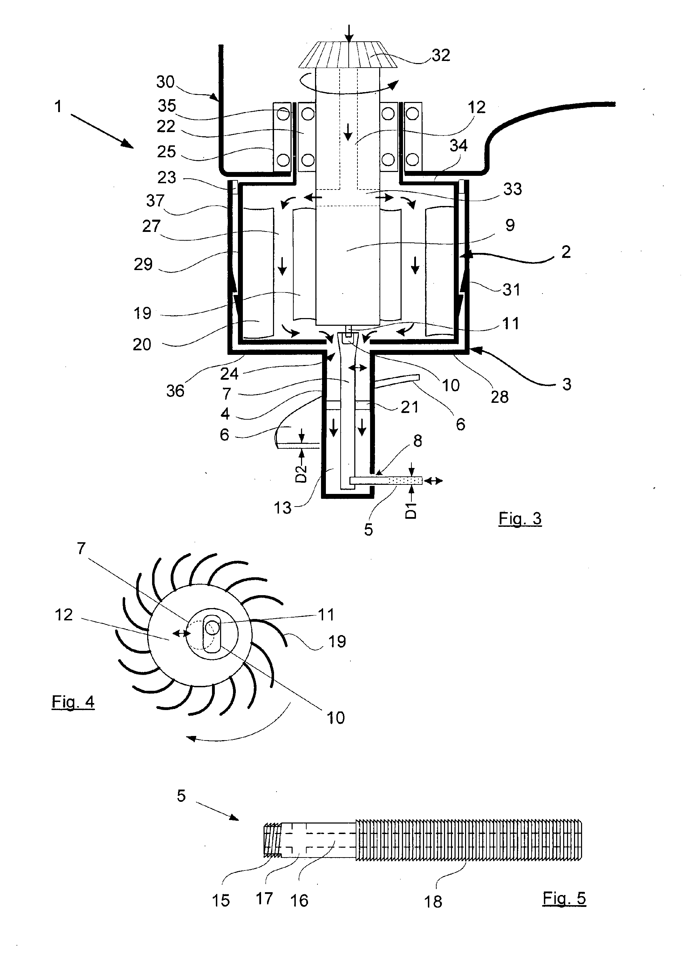

Figur 1 zeigt ein kieferchirurgisches Werkzeug 1 zur Einbringung einer helikalen Kavität in einen Kieferknochen, der hier der Einfachheit halber nicht dargestellt ist. Das Werkzeug 1 weist ein Hauptteil 2 und ein Werkzeugteil 3 auf. In dem Hauptteil 2 liegt eine Antriebswelle 9 zumindest teilweise ein, siehe Figur 3. Das Werkzeugteil 3 ist an dem Hauptteil gehalten. Es umfasst einen drehbaren Schaft 4, der koaxial vom Hauptteil 2 hervorsteht. An dessen dem Hauptteil 2 gegenüberliegenden Ende besitzt der Schaft 4 eine Öffnung 8, durch die sich ein Stift 5 hindurch erstreckt. Der Stift 5 ist das eigentliche Werkzeug und in Richtung seiner Längsachse beweglich. Er ist mit einem Ende einer Vibrationswelle 7 verbunden, die sich durch den Schaft 4 hindurch erstreckt und an deren anderem Ende Vibrationen eingekoppelt oder erzeugt werden, die sich entlang der Vibrationswelle derart übertragen, dass der Stift in Längsrichtung oszilliert. Die Oberfläche des Stifts 5 ist gemäß einer ersten Ausführungsvariante zumindest in dem aus dem Schaft 4 herausragenden Bereich diamantiert, so dass er in Folge der Oszillationsbewegung wie eine Feile wirkt.

Vom Außenmantel des Schafts 4 steht ein Flügel 6 radial ab, der sich helikal in Umfangsrichtung des Schafts 4 erstreckt. Figur 2 zeigt eine Ansicht des erfindungsgemäßen kieferchirurgischen Werkzeugs 1 von unten. An dieser Darstellung wird deutlich, dass die Stirnkante 14 des Flügels 6 in einem Abstand hinter dem Stift 5 liegt und der Flügel 6 sich bezogen auf die Schaftachse auf derselben Höhe wie der Stift 5 beginnend in helikaler Richtung entlang des Außenumfangs des Schafts 4 nach oben erstreckt, d.h. sich schraubenlinienförmig um den Schaftaußenmantel herum windet. Dabei vollführt der Flügel 6 keine vollständige Drehung sondern erstreckt sich nur entlang eines etwa 4/5 Kreises.

Der Flügel 6 ist nicht selbstschneidend. Seine radial außen liegende Kante ist stumpf ausgebildet. Die Dicke D2 des Flügels 6 (siehe Figur 3) in Richtung einer auf dem Flügel 6 stehenden Normalen beträgt etwa 1 mm. Der Stift 5 ragt geringfügig weiter aus dem Schaft 4 hinaus als die radiale Breite des Flügels 6 ist, so dass der Flügel 6 die Knochenwand der Kavität nicht berührt.

Der Schaft 4 ist drehbar mit der Antriebswelle 9 gekoppelt, um ein Drehmoment auf den Stift 5, d.h. einen Vorschub in Umfangsrichtung auszuüben, wie der Pfeil in Figur 1 andeutet. Die Drehung ist dabei derart, dass der Stift 5 in der Drehungsrichtung vorne liegt und sich der Flügel 6 in der Art eines Schweifs hinter dem Stift 5 befindet.

Figur 3 zeigt eine Detaildarstellung eines Beispiels des erfindungsgemäßen Werkzeugs 1 in einer Schnittdarstellung. Es sei angemerkt, dass es sich hierbei lediglich um eine Prinzipskizze handelt, um die Funktionsweise des erfindungsgemäßen Werkzeugs 1 zu veranschaulichen. Technische Details, die die Funktionsfähigkeit des Werkzeugs gewährleisten, bei beispielsweise Haltemittel oder Dichtungen sind aus Darstellungsgründen zum Teil nicht gezeigt. Die tatsächliche Ausgestaltung des Werkzeugs kann sich ferner von der Darstellung in Figur 3 unterscheiden, ohne dabei vom erfindungsgemäßen Grundgedanken abzuweichen.

Das Werkzeug 1 ist in einem in der Zahnmedizin hinlänglich bekannten Winkelstück 30 gehalten und dort drehbar gelagert. Zur Lagerung dient ein erstes Lager 25, hier beispielhaft als Kugellager ausgebildet, in das das Hauptteil 2 eingesetzt ist. In dem Winkelstück 30 ist eine nicht dargestellte angetriebene Welle vorhanden, in die ein endseitig einer Antriebswelle 9 des Werkzeugs 1 vorgesehenes Kegelrad 32 eingreift. Eine Drehung der Welle im Winkelstück 30 wird folglich durch das Kegelrad 32 auf die Antriebswelle 9 des Werkzeugs 1 übertragen.

Das Hauptteil 2 des Werkzeugs 1 ist in diesem Ausführungsbeispiel trommelförmig ausgebildet. Es besitzt ein rohrförmiges Gehäuse 29, das an seinen Endseiten durch jeweils eine Kreisringscheibe 34, 36 zumindest teilweise geschlossen ist. Winkelstückseitig schließt sich an die dortige Kreisringscheibe 34 ein rohrförmiger Kragen 35 an, der konzentrisch zum Gehäuse 29 liegt und fest mit der Kreisringscheibe 34 verbunden ist. Das Gehäuse 29, der Kragen 35 und die beiden Kreisringscheiben 34, 36 können einstückig als ein einziges Bauteil, beispielsweise aus Metall gefertigt sein. Innerhalb des Kragens 35 liegt ein weiteres Lager 22 ein, hier ebenfalls beispielhaft ein Kugellager, in dem die Antriebswelle 9 gelagert ist. Außenseitig liegt der Kragen 35 an dem ersten Lager 25 an, wodurch das Hauptteil 2 am Winkelstück 30 drehbar gelagert ist.

Die Antriebswelle 9 erstreckt sich durch das zweite Lager 22 hindurch in das Hauptteilgehäuse 29 hinein. An dem dem Winkelstück 30 abgewandten Endbereich weist die Antriebswelle 9 an ihrem Außenumfang eine Vielzahl radial abstehender Lamellen 19 auf. Diese sind entlang des Außenumfangs der Antriebswelle 9 äquidistant angeordnet und erstreckenden sich in Drehrichtung der Antriebswelle 9 bogenförmig nach außen. Dies wird durch Figur 4 veranschaulicht, die einen Blick von unten auf die Antriebswelle 9 zeigt.

An der Innenseite des Hauptteilgehäuses 29 sind ebenfalls eine Vielzahl Lamellen 20 äquidistant angeordnet, die sich im Wesentlichen bogenförmig radial nach innen erstrecken, wobei ihre bogenförmige Erstreckung derjenigen der Lamellen19 der Antriebswelle 9 entgegengesetzt ist. Die Lamellen 19 der Antriebswelle und die Lamellen 20 des Hauptteilgehäuses 29 liegen beabstandet zueinander, wobei zwischen Ihnen ein Freiraum 27 gebildet ist, das von einem Fluid gefüllt ist. Dieses Fluid kann ein Gas wie beispielsweise Luft sein. Die Lamellen 19 der Antriebswelle 9 wirken im Falle ihrer Drehung wie ein Lüfterrad und erzeugen im Hauptteilgehäuse 29 eine Art Zyklon drehender Luft. Dieser bewirkt auf die konkave Innenseite der Lamellen 20 des Hauptteilgehäuses 29 eine Druckkraft, die in einem Drehmoment um die Drehachse des Hauptteils 2 resultiert. Zwischen der Antriebswelle 9 und dem Hauptteil 2 ist auf diese Weise eine hydrodynamische Kupplung gebildet. Durch die Verwendung einer Flüssigkeit, beispielsweise Wasser, wird diese Kraftübertragung noch stärker. Die Höhe der Kraftübertragung kann neben der Art des Fluids durch die Anzahl und Länge der Lamellen 19, 20 beeinflusst werden.

Das Gehäuse 29 des Hauptteils 2 ist von dem Werkzeugteil 3 umgriffen. In der Ausführungsvariante gemäß Figur 3 ist das Werkzeugteil 3 ebenfalls trommelförmig. Es weist ein rohrförmiges Gehäuse 37 größeren Durchmessers als das Gehäuse 29 des Hauptteils 2 auf, das das Hauptteilgehäuse 29 umfänglich umgreift. Das Werkzeugteil 3 ist ein Verschleißteil und kann bei Bedarf ausgewechselt werden. Hierzu ist das Werkzeugteil 3 lösbar auf das Hauptteil 2 gesteckt. Das Zusammenfügen erfolgt in koaxialer Richtung zueinander, beispielsweise mittels einer Rastverbindung oder mittels Bajonettverbindung. In Figur 3 sind Rastmittel 31 zu erkennen, die eine Befestigung des Werkzeugteilgehäuses 37 am Hauptteilgehäuse 29 in axialer Richtung gewährleisten.

Das rohrförmige Gehäuse 37 des Werkzeugteils 3 ist an dem dem Winkelstück 30 abgewandten Ende durch eine konzentrische Kreisringscheibe 28 geschlossen, die eine zentrale Öffnung 24 aufweist. Konzentrisch zu besagter Kreisringscheibe 28 umgibt ein hohler Schaft 4 die Öffnung 24, der mit der Kreisringscheibe 28 fest verbunden ist. Auch hier können das Gehäuse 37, die Kreisringscheibe 28 und der Schaft 4 einstückig als ein einziges Bauteil, beispielsweise aus Metall gefertigt sein.

In dem Schaft 4 ist eine Vibrationswelle 7 angeordnet, die durch einen Stab gebildet ist und sich koaxial im Schaft 4 bis nahe an das untere Schaftende erstreckt. Sie ist durch eine Festlagerscheibe 21 gehalten. An dem dem Hauptteil 2 abgewandten Ende der Vibrationswelle 7 ist der Stift 5 befestigt, der in der Ausführung gemäß Figur 3 auf der Oberfläche seines aus dem Schaft 4 heraus ragenden Bereichs diamantiert ist. Die Vibrationswelle 7 ragt an ihrem gegenüberliegenden Ende durch die Öffnung 24 in der Kreisringscheibe 28 sowie durch eine entsprechende Öffnung in der Kreisringscheibe 36 des Hauptteils 2 hindurch und in das Hauptteil 2 hinein. An diesem Ende ist sie mit der Antriebswelle 9 gekoppelt.

Diese Kopplung erfolgt durch eine in der Stirnseite der Vibrationswelle 7 vorhandene Langnut 10, in die ein von der Stirnseite der Antriebswelle 9 abstehender Exzenterstift 11 hineinragt. Der Exzenterstift 11 ist zur Drehachse der Antriebswelle leicht versetzt, so dass er sich bei einer Drehung der Antriebswelle 9 auf einer Kreisbahn bewegt. Diese Kreisbahn ist in Figur 4 gestrichelt dargestellt.

Figur 4 zeigt eine Schnittansicht eines Radialschnitts durch das Ende der Vibrationswelle in Höhe der Langnut 10 und Exzenterstift 11 mit Blick auf die Unterseite der Antriebswelle 9. Entlang seiner Kreisbahn bewegt sich der Exzenterstift 11 innerhalb der Langnut 10 hin und her. Er drückt dabei abwechselnd gegen die Längsseiten der Langnut 10 und nimmt die Vibrationswelle 7 auf seiner Kreisbahn mit, so dass diese eine oszillierende Bewegung quer zur Längserstreckung der Langnut 10 vollführt. Diese Bewegung ist durch den Doppelpfeil in Figur 4 verdeutlicht. Die Antriebswelle 9 kann mit einer Drehzahl zwischen 20.000 U/min und 40.000 U/min drehen. Entsprechend ergibt sich eine Oszillation im Bereich zwischen 50 kHz und 600 Hz. Die Amplitude der Oszillation kann durch den Abstand des Exzenterstifts 11 von der Drehachse der Antriebswelle 9 festgelegt werden.

Die endseitig erzeugten Schwingungen pflanzen sich entlang der Vibrationswelle 7 vom Einkopplungsort zum gegenüberliegenden Ende fort, an dem sich der Stift 5 befindet. Etwa mittig zwischen dem Exzenterstift 11 und dem Stift 5 ist das Festlagerscheibe 21 in Gestalt einer Scheibe vorhanden, durch das sich die Vibrationswelle 7 konzentrisch hindurch erstreckt. Durch diese Festlagerscheibe 21, gegen die sich die Vibrationswelle 7 abstützt, werden Schwingungen der Welle 7 an dieser Stelle unterdrückt und die Vibrationswelle 7 wird innerhalb des Schafts 4 stabilisiert. Gleichwohl setzen sich die Schwingungen hinter der Festlagerscheibe 21 fort, so dass der Stift 5 entlang seiner Längsachse wie durch den Doppelpfeil in Figur 3 angedeutet in Längsrichtung schwingt. Aufgrund der Diamantierung wirkt der Stift 5 wie eine Feile und feilt Knochenmaterial im Kieferknochen weg.

Gemäß einer nicht dargestellten Alternative zur Festlagerscheibe 21, kann sich die Vibrationswelle 7 mittels einer schwenkbaren Lagerung im Schaft 4 abstützen. Diese Lagerung kann beispielsweise dadurch realisiert sein, dass von der Vibrationswelle 7 zwei sich gegenüberliegende Zapfen abstehen, die in entsprechenden Aufnahmen innenseitig des Schafts schwenkbar gelagert einliegen. Anstelle dieser in ihrer Form und Größe an die Zapfen angepassten Aufnahmen, kann der Schaft 4 innenseitig eine einfache ringförmige Stufe aufweisen, auf der die Zapfen aufliegen. Vorteilhafterweise ist zusätzlich eine konzentrische Hülse aus Richtung der Antriebswelle in den Schaft hineingeschoben und reicht in axialer Richtung bis nahe an die Zapfen heran, so dass sie auf der dieser Stufe gegenüberliegenden Seite der Zapfen angeordnet ist und die Vibrationswelle 7 in ihrer Bewegungsfreiheit zusätzlich einschränken kann. Die beiden Zapfen bilden eine Achse, um die die Vibrationswelle 7 während der Oszillation geschwenkt wird.

Der Stift 5 besitzt eine Dicke D1 die größer ist als die Dicke D2 des Flügels 6. Bei einer Dicke D2 des Flügels 6 von 1 mm kann die Dicke D1 des Stifts 5 beispielsweise 1,2 mm betragen. Der Stift 5 kann einen runden oder ovalen Querschnitt aufweisen, wobei im Falle eines ovalen Querschnitts die Schmalseite des Stifts 5 in Umfangsrichtung des Schafts 4 orientiert ist, d.h. in Richtung der Drehbewegung des Schafts.

Wie bereits erwähnt, ist der Schaft 4 drehbar. Dies erfolgt dadurch, dass der Schaft fest mit dem Gehäuse 37 des Werkzeugteils 3 und dieses wiederum drehfest mit dem Gehäuse 29 des Hauptteils 2 verbunden ist. Eine drehfeste Verbindung zwischen den Gehäusen 29, 37 von Hauptteil 2 und Werkzeugteil 3 kann beispielsweise durch radiale Vorsprünge und / oder Vertiefungen erreicht werden, die in Umfangsrichtung miteinander zumindest einen Anschlag, vorzugsweise mehrere Anschläge bilden.

Es ist von Vorteil, wenn aus dem Schaft 4 eine Flüssigkeit strömt, die zur Spülung der vom Stift 5 erzeugten Kavität dient. In Folge der Spülung werden von dem Stift 5 abgeriebene Knochenpartikel aus der Kavität heraus geschwemmt. Die diamantierte Oberfläche des Stifts 5 wird zudem gereinigt so dass die Feilwirkung des Stifts erhalten bleibt. Um dies zu ermöglichen, ist das gesamte erfindungsgemäße Werkzeug 1 flüssigkeitsdurchströmt, was durch entsprechende Pfeile in Figur 3 angedeutet ist. Die Flüssigkeit, beispielsweise sterilisiertes Wasser, wird in das Winkelstück 30 eingeleitet. Die Antriebswelle 9 ist zumindest teilweise als Hohlwelle ausgebildet und besitzt einen inneren Kanal 12, der sich vom winkelstückseitigen Ende der Antriebswelle 9 bis in das Hauptteil 2 hinein erstreckt. Der Kanal 12 ist endseitig der Antriebswelle 9 offen, so dass die Flüssigkeit im Winkelstück 30 dort eintreten kann.

Im Bereich innerhalb des Hauptteils 2 sind eine oder mehrere Querbohrungen 33 in der Antriebswelle 9 vorgesehen, die den Kanal 12 mit dem Innenraum 27 des Hauptteils 2 verbinden. Die Flüssigkeit kann somit durch diese Querbohrungen 33 aus- und in den Innenraum 27 eintreten, wo es als Drehmomentübertragungsmedium der hydrodynamischen Kupplung dient.

Auf der dem Winkelstück 30 gegenüberliegenden Seite des Hauptteils 2 strömt die Flüssigkeit durch die zentrale Öffnung der Kreisringscheibe 36 sowie durch die Öffnung 24 der weiteren Kreisringscheibe 28 des Werkzeugteils 3 und tritt dann in den Innenraum 13 des Schafts 4 ein, wo sie parallel zur Vibrationswelle 7 fließt. Eine Dichtung 23 zwischen dem Hauptteilgehäuse 29 und dem Werkzeugteilgehäuse 37 verhindert, dass Flüssigkeit aus dem Spalt zwischen Hauptteil 2 und Werkzeugteil 3 austritt.

Um den Austritt der Flüssigkeit am Ende des Schafts 4 zu ermöglichen, kann die Stirnkante 26 uneben ausgebildet sein, wie dies in Figur 1 dargestellt ist. Gemäß dieser Ausführungsvariante besitzt sie zwei einander gegenüberliegende Vorsprünge, die durch einen in Umfangsrichtung etwa sinusförmigen Verlauf der Stirnkante 26 gebildet sind. Die Vorsprünge bewirken, dass die Stirnkante 26 des Schafts 4 nicht vollflächig am Boden der Bohrung im Kieferknochen aufliegt. So halten die Vorsprünge den Bereich der Stirnkante 26, der zwischen den Vorsprüngen liegt, beabstandet zum Boden der Bohrung, so dass die Flüssigkeit unter der Stirnkante 26 aus dem Schaft 4 heraustreten kann.

Die Stirnseite des Schafts 4 kann jedoch auch eben ausgebildet sein, wie dies in Figur 3 zu sehen ist. Die Flüssigkeit kann dann alternativ oder zusätzlich durch den ringförmigen Spalt in der Öffnung 8 zwischen dem Stift 5 und dem Schaft 4 austreten.

Wie in Figur 5 gezeigt, ist zusätzlich vorgesehen, dass sich in Längsrichtung des Stifts 5 ein Kanal 16 erstreckt, der zu beiden Axialseiten des Stifts 5 offen ist. Die Flüssigkeit kann in diesen Kanal 16 durch sich radial erstreckende Querbohrungen 17 eintreten. Die Flüssigkeit tritt dann aus dem der Vibrationswelle 7 abgewandten Ende des Stifts 5 heraus und strömt von dort durch die gesamte erzeugte Kavität.

Wie aus Figur 5 ferner zu entnehmen ist, besitzt der Stift 5 an dem der Vibrationswelle 7 zugewandten Ende ein Gewinde 15, mit dem er in die Welle 7 einschraubbar ist. Dies ermöglicht einen Wechsel des Stifts 5 im Verschleißfalle.

Des Weiteren zeigt Figur 4 eine zur Diamantierung alternative Oberfläche des Stifts 5. Sein aus dem Schaft 4 hervorstehender Teil besitzt gemäß dieser Ausführungsvariante ein rotationssymmetrisches Sägezahnprofil 18. Aufgrund dieses Profils 18 sorgt eine Oszillationsbewegung in Längsrichtung des Stifts 5 nicht für einen feilenden sondern für einen sägenden Abrieb von Knochenmaterial. Dies hat den Vorteil, dass die helikale Kavität deutlich schneller gebildet werden kann.

Es sei angemerkt, dass zur Anwendung des erfindungsgemäßen Werkzeugs 1 eine Bohrung im Kieferknochen Voraussetzung ist, deren Innendurchmesser mindestens dem Außendurchmessers des Schafts 4 entsprechen sollte, damit der untere Bereich des Schafts 4 in diese Bohrung eingesetzt werden kann, bis der Stift 5 Kontakt mit der Oberseite des Kieferknochens erhält. Sodann wird die Antriebswelle 9 aktiviert, wodurch der Stift 5 eine Oszillationsbewegung in seiner Längsrichtung vollführt und Knochenmaterial abträgt. Gleichzeitig wird von der Antriebswelle durch die hydrodynamische Kupplung ein Drehmoment auf das Werkzeugteil 3, den Schaft 4 und den Stift 5 ausgeübt, so dass der Stift 5 unter Kontakt mit dem Knochen arbeitet und mit Fortschritt der Kavitätsbildung langsam eine Drehbewegung ausführt. Durch die Überlagerung dieser beiden Bewegungen arbeitet sich der Stift 5 schräg in den Kieferknochen hinein.

Sobald sich der Stift 5 zwei bis drei Millimeter auf einem Umfangswinkel von etwa 90° in den Kieferknochen hinein gearbeitet hat, ragt der Flügel 6 in die so hergestellte Kavität bereits hinein, wobei mit zunehmendem Fortschritt der Kavitätsherstellung der Flügel 6 weiter in diese Kavität hineinreicht, insbesondere fast formschlüssig. Dies stabilisiert das Werkzeug 1 und verhindert ein Verkippen, so dass die Achse der helikalen Kavität koaxial zur Bohrung liegt und den Implantatkörper eines Zahnimplantats mit seinem entsprechend helikalen Flügel zur Verankerung des Implantats formschlüssig aufnehmen kann.

Auf diese Weise wird im Kieferknochen nur ein Minimum an Knochenmaterial entfernt, so dass der Heilungsprozess, während dessen der Kieferknochen mit dem Zahnimplantat verwächst, erheblich verkürzt wird.

### Bezugszeichenliste

- 1: Chirurgisches Werkzeug
- 2: Hauptteil
- 3: Werkzeugteil
- 4: Schaft
- 5: Stift
- 6: Flügel
- 7: Vibrationswelle
- 8: Öffnung im Schaft
- 9: Antriebswelle
- 10: Langnut
- 11: Exzenterstift
- 12: Kanal
- 13: Innenraum des Schafts
- 14: Stirnkante des Flügels
- 15: Gewinde
- 16: Kanal
- 17: Querbohrung
- 18: Sägezahnprofil
- 19: Außenlamellen
- 20: Innenlamellen
- 21: Festlagerscheibe
- 22: Drehlager
- 23: Dichtring
- 24: Öffnung in Werkzeuggehäuse
- 25: Drehlager
- 26: Stirnkante des Schaft
- 27: Innenraum des Hauptteils/ Freiraum
- 28: Kreisringscheibe
- 29: Gehäuse des Hauptteils 2
- 30: Winkelstück
- 31: Haltemittel
- 32: Kegelrad
- 33: Querbohrungen
- 34: Kreisringscheibe
- 35: Kragen
- 36: Kreisringscheibe
- 37: Gehäuse des Werkzeugteils 3

## Patentansprüche

1. Chirurgisches Werkzeug (1) zur Einbringung einer helikalen Kavität in einen Knochen mit einem zumindest einen Teil einer Antriebswelle (9) aufnehmenden Hauptteil (2) und einem an diesem gehaltenen Werkzeugteil (3), welches ein Funktionselement (5) umfasst, das durch Ausübung einer oszillierenden Bewegung die Kavität im Knochen erzeugt, wobei das Werkzeugteil (3) einen abstehenden, drehbaren Schaft (4) umfasst, an dessen dem Hauptteil (2) gegenüberliegenden Ende sich ein das Funktionselement bildender Stift (5) durch eine Öffnung (8) aus dem Schaft (4) heraus erstreckt, der zur Ausübung einer Materialabtragung des Knochens in seiner Längsrichtung oszillierbar ist, und dass der Schaft (4) derart mit der Antriebswelle (9) gekoppelt ist, dass diese zumindest mittelbar ein Drehmoment auf den Schaft (4) und den Stift (5) ausübt, und wobei vom Schaft (4) zumindest ein Flügel (6) radial absteht, der in einem Abstand hinter dem Stift (5) beginnend sich in Umfangsrichtung des Schafts (4) helikal in Richtung des Hauptteils (2) erstreckt, **dadurch gekennzeichnet, dass** die Kopplung des Schaftes (4) mit der Antriebswelle (9) über eine hydrodynamische Kupplung erfolgt.

2. Chirurgisches Werkzeug (1) nach Anspruch 1, **dadurch gekennzeichnet, dass** der Stift (5) außenseitig diamantiert ist oder ein Sägezahnprofil aufweist.

3. Chirurgisches Werkzeug (1) nach Anspruch 1 oder 2, **dadurch gekennzeichnet, dass** sich der Flügel (6) entlang 2/3 bis 4/5 des Schaftumfangs erstreckt.

4. Chirurgisches Werkzeug (1) nach einem der Ansprüche 1 bis 3, **dadurch gekennzeichnet, dass** der Flügel (6) in radialer Richtung eine Breite aufweist, die das 0,5 bis 1,2-fache des Durchmessers des Schafts (4) besitzt.

5. Chirurgisches Werkzeug (1) nach einem der vorherigen Ansprüche, **dadurch gekennzeichnet, dass** die Antriebswelle (9) einen von ihrem stirnseitigen Eende abstehenden Exzenterstift (11) aufweist, der mit einer sich durch den Schaft (4) erstreckenden Vibrationswelle (7) in Eingriff steht, wobei der Stift (5) an der Vibrationswelle (7) fest gehalten ist.

6. Chirurgisches Werkzeug (1) nach Anspruch 5, **dadurch gekennzeichnet, dass** sich die Vibrationswelle (7) an einer Festlagerscheibe (21) abstützt, die vorzugsweise etwa mittig im Schaft (4) angeordnet ist.

7. Chirurgisches Werkzeug (1) nach einem der vorherigen Ansprüche, **dadurch gekennzeichnet, dass** sich dem Stift (5) diametral gegenüberliegend ein zweiter Stift durch eine weitere Öffnung aus dem Schaft (4) heraus erstreckt, der, insbesondere im Gegentakt zum ersten Stift (5), zur Ausübung einer Feilwirkung in Längsrichtung oszillierbar ist.

8. Chirurgisches Werkzeug (1) nach Anspruch 7, **dadurch gekennzeichnet, dass** vom Schaft (4) ein zweiter Flügel radial absteht, der sich in einem Abstand hinter dem zweiten Stift beginnend in Umfangsrichtung des Schafts (4) helikal in Richtung des Hauptteils (2) erstreckt.

9. Chirurgisches Werkzeug (1) nach einem der vorherigen Ansprüche, **dadurch gekennzeichnet, dass** neben dem Stift (5) auf der dem Flügel (6) gegenüberliegenden Seite in derselben Radialebene wie der Stift (5) eine Öffnung im Schaft (4) vorhanden ist.
